# EUROPÄISCHE PATENTSCHRIFT

(11) **EP 0 249 008 B1**
(45) Veröffentlichungstag und Bekanntmachung des Hinweises auf die Patenterteilung: **15.09.1993**
(21) Anmeldenummer: 87105548.9
(22) Anmeldetag: 14.04.1987
(51) Int. Cl.: A61K 31/70

(54) **Verwendung von spezifischen Monosacchariden zur Herstellung eines Arzneimittels zur Verhinderung von Metastasen maligner Tumore**
Use of specific monosaccharides for the preparation of a medicament for the prevention of metastases of malignic tumours
Utilisation de monosaccharides spécifiques pour la préparation d'un médicament pour la prévention des métastases de tumeurs malignes

(30) Priorität: 09.05.1986 DE 3615621
(43) Veröffentlichungstag der Anmeldung: 16.12.1987
(73) Patentinhaber: Pulverer, Gerhard, Prof. Dr.Dr.h.c., 50858 Köln (DE); Oette, Kurt, Prof. Dr. med., 50933 Köln (DE); Uhlenbruck, Gerd, Prof. Dr. med., D-50935 Köln (DE)
(72) Erfinder: Pulverer, Gerhard, Prof. Dr.Dr.h.c., 50858 Köln (DE); Oette, Kurt, Prof. Dr. med., 50933 Köln (DE); Uhlenbruck, Gerd, Prof. Dr. med., D-50935 Köln (DE)
(74) Vertreter: Werner, Hans-Karsten, Dr.Dipl.-Chem.

(56) Entgegenhaltungen:
- EP-A- 0 136 438
- EP-A- 0 157 427
- CA-A- 1 120 399
- GB-A- 1 158 456
- GB-A- 2 106 935
- Chemical Abstracts, vol. 80, no. 21, May 27, 1974, p. 190, abstract 117838x, Columbus, Ohio, US A.S. Evans et al.
- Chemical Abstracts, vol. 102, no. 25, June 24, 1985, p. 436 abstract 219087s, Columbus, Ohio, US S.C. Barnett et al.

## Beschreibung

Die Bildung von Metastasen maligner Tumore, ausgehend von einem primären Tumor an mehr oder Weniger entfernten Stellen des Körpers, ist eines der gewichtigsten Probleme der Tumortherapie, da die meisten tödlichen Erkrankungen auf derartige Metastasen zurückzuführen sind. Die Behandlung primärer Tumore durch Chirurgie, Bestrahlung und Chemotherapie hat in den letzten Jahren erhebliche Erfolge aufzuweisen. Die Behandlung von Metastasen ist hingegen äußerst schwierig und nur in den wenigsten Fällen erfolgreich. Insbesondere während der Behandlung von Primärtumoren ist das Risiko der Metastasenbildung besonders hoch, so daß ein dringendes Bedürfnis danach besteht, gerade in dieser Phase die Bildung von Metastasen zu verhindern. Intensive Untersuchungen der Bildung von Metastasen, und zwar organspezifischen und nicht organspezifischen Metastasen, haben zu dem Ergebnis geführt, daß für die Bildung von Metastasen Organzell-Lektine verantwortlich sind. Lektine sind hochspezifische zuckerbindende Moleküle, welche zunächst nur in Pflanzen, später aber auch in nahezu allen anderen Lebewesen bis hin zu Vertebraten gefunden wurden. Die Lektine dienen anscheinend in der Hauptsache der Erkennung von Zuckerstrukturen an Zelloberflächen oder in löslichen Glykokonjugaten.

Es wurde weiterhin gefunden, daß Organzell-Lektine für die spezifisch organotrope Metastasierung verantwortlich sind. So beschreiben die Autoren Barnett und Eccles in ihrer Arbeit Clin. Exp. Metastasis, 1984, Vol. 2, Seiten 297 bis 310, daß ihre in vitro gezeigte Lektin-spezifische Anlagerung fusiformer Mammakarzinom-Zellen an die Leberzellen der Maus in vivo einer von vielen Faktoren sein könnte, der für die Entstehung von Metastasen wichtig ist.

Durch intensive Untersuchungen der Erfinder wurde gefunden, daß die Bildung von Metastasen maligner Tumore verhindert werden kann, wenn man diese Organzell-Lektine mit den für sie spezifischen Monosacchariden und/oder endständig diese Monosaccharide enthaltenden Glykokonjugaten absättigt. Von besonderer Bedeutung sind dabei die β-D-Galactose und/oder endständige β-D-Galactose enthaltende Glykokonjugate. Weitere bedeutungsvolle Monosaccharide sind die Mannose sowie endständige oder mittelständige Mannose enthaltende Glykokonjugate sowie L-Fukose, N-Acetylglukosamin, N-Acetyl-Galactosamin, N-Acyl-Neuraminsäuren und Neuraminsäurehaltige Derivate. Die Glykokonjugate sind somit Substanzen, die das spezifische Monosaccharid endständig oder mittelständig gebunden haben an ein pharmakologisch inertes Trägermolekül. Dieses Trägermolekül soll zumindest nicht selbst cytotoxisch gegen Tumorzellen wirksam sein. Typische Trägermoleküle sind somit im einfachsten Fall andere Zucker, so daß erfindungsgemäß außer den spezifischen Monosacchariden selbst auch diese enthaltende Disaccharide, Trisaccharide und Oligosaccharide eingesetzt werden können. Vorzugsweise sollen die Glykokonjugate nicht oder nur schwach immunogen sein, um nicht zu Nebenwirkungen zu führen oder durch die Immunantwort unwirksam gemacht zu werden.

In den die spezifischen Monosaccharide enthaltenden Glykokonjugaten sollten sie vorzugsweise endständig gebunden sein. Es hat sich jedoch gezeigt, daß auch mittelständig gebundene Monosaccharide wirksam sind. Hierbei ist jedoch noch nicht geklärt, ob in diesen Glykokonjugaten der eine oder der andere Rest so leicht abgespalten wird, daß in vivo rasch Glykokonjugate mit einem endständigen spezifischen Monosaccharidrest entstehen.

So konnte jetzt gezeigt werden, daß beispielsweise das Dickdarmkarzinom dazu neigt, Metastasen in der Leber zu bilden. Es konnte weiter gezeigt werden, daß durch Verabreichung von Galactose oder Galactose enthaltenden Glykokonjugaten, wie Arabinogalactan, die Metastasierung völlig unterbunden werden kann. Durch Vergleichsversuche mit Mannan als Galactosefreier Kontrollsubstanz konnte gezeigt werden, daß das gleiche Metastasierungsverhalten wie bei einer unbehandelten Kontrollgruppe bestehen bleibt. Die Applikation von Galactose oder Arabinogalactan ist nur nötig für eine relativ kurze Frist vor und nach der Behandlung des Primärtumors bzw. kurz vor und nach potentiell zur Metastasierung führenden diagnostischen Eingriffen bei Tumorverdacht. Sofern jedoch keine Nebenwirkungen zu befürchten sind, können die Monosaccharide und/oder diese Monosaccharide enthaltende Glykokonjugate von der Diagnose des Tumors ab bis zu einigen Wochen nach der Therapie appliziert werden. Die Behandlung mit den für Organzell-Lektine spezifischen Monosacchariden und/oder diese Monosaccharide enthaltenden Glykokonjugaten ist selbstverständlich nur wirksam bezüglich der Verhinderung der organspezifischen Ansiedlung von Metastasen. Anscheinend ist diese organspezifische Metastasierung aber besonders häufig und daher besonders gefährlich. Die organspezifische Metastasierung unterdrücken zu können, ist somit bereits ein wesentlicher therapeutischer Erfolg.

Gegenstand der Erfindung ist somit die Verwendung von für Organzell-Lektine spezifischen Monosacchariden und/oder diese Monosaccharide enthaltenden Glykokonjugaten zur Herstellung von Arzneimitteln zur Verhinderung der organspezifischen Ansiedlüng von Metastasen maligner Tumore, wobei das Monosaccharid β-D-Galactose, L-Fükose, Mannose, N-Acetyl-Glükosamin, N-Acetyl-Galactosamin, N-Acyl-Neuraminsäure oder ein Gemisch derselben ist.

Insbesondere wenn es sich um Tumore handelt, die dazu neigen, organspezifische Metastasen zu bilden, kann jetzt gezielt geprüft werden, ob die hierfür verantwortlichen Organzell-Lektine durch Applikation von entsprechendem Monosaccharid und/oder diese Monosaccharide enthaltenden Glykokonjugaten blockiert werden können.

Da Galactose und Galactose enthaltende Glykokonjugate bekannt sind und ausgesprochen gut verträglich sind, kann die neue Therapie relativ einfach und problemlos durchgeführt werden. Diese Wirkstoffe können sowohl enteral als auch parenteral appliziert werden. Sie werden in bekannter Weise metabolisiert oder aus dem Körper ausgeschieden. Der relativ hohe Serumspiegel an Galactose reicht aber offensichtlich völlig aus, die entscheidenden Organzell-Lektine so lange zu blockieren wie erhöhte Metastasierungsgefahr durch Behandlung des Primärtumors besteht. Das gleiche gilt selbstverständlich auch für die Phase diagnostischer Eingriffe, bei denen leicht Tumorzellen in die Blutbahn gelangen können. Die Versuchsergebnisse deuten darauf hin, daß die monoklonalen Antikörper gegen die Organzell-Lektine in ähnlicher Weise die Metastasierung unterbinden, wie die jetzt auch als hochwirksam gefundenen spezifischen Monosaccharide und/oder vorzugsweise endständig diese Monosaccharide enthaltenden Glykokonjugate. Es ist einleuchtend, daß die erfindungsgemäßen Mittel wesentlich einfacher, preiswerter und sicherer zu handhaben sind als die nur relativ schwierig und kostspielig herstellbaren monoklonalen Antikörper.

Aus umfangreichen Tierexperimenten und inzwischen erfolgreichen klinischen Untersuchungen ergibt sich beispielsweise, daß bei Applikation von 120-150 g Galactose pro Tag bei diagnostiziertem Dickdarmkarzinom ab dem Zeitpunkt der Diagnose bis 4 Wochen nach der erfolgreichen Therapie keine Leber-Metastasen mehr beobachtet wurden, obwohl diese Metastasen ohne die erfindungsgemäße Applikation von Galactose statistisch sehr häufig beobachtet werden. Die applizierten Mengen an Galactose werden nebenwirkungsfrei über längere Zeit vertragen. Tierexperimente haben gezeigt, daß auch Arabinogalactan wirksam ist. Neuraminspezifische Organ-Lektine konnten mit Neuraminidase behandelten Bakterienpräparaten aus Streptokokken und Propionibakterien erfolgreich blockiert werden.
Als Arabinogalactan wurde eingesetzt ein Präparat aus Larix europaea mit einem mittleren Molekulargewicht von 70.000 (Serva GmbH, Heidelberg). Es handelt sich um ein nicht immunogenes Oligosaccharid, welches auch als Plasmaexpander eingesetzt werden kann.

Tierexperimente mit Galaktan aus Gummiarabicum, Dextran und Mannan sowie anderen Mono- und Disacchariden haben gezeigt, daß diese nicht in der Lage sind, die für Galaktose spezifischen Lektine der Leber zu blockieren und damit die Ansiedlung von Metastasen in der Leber zu unterdrücken.

## Patentansprüche

1. Verwendung von für Organzell-Lektine spezifischen Monosacchariden und/oder monosaccharidhaltigen Glykokonjugaten, wobei das Monosaccharid β-D-Galactose, L-Fukose, Mannose, N-Acetyl-Glukosamin, N-Acetyl-Galactosamin, N-Acyl-Neuraminsäure oder ein Gemisch derselben ist, zur Herstellung eines Arzneimittels zur Verhinderung der organspezifischen Ansiedlung von Metastasen maligner Tumore.

2. Verwendung nach Anspruch 1, dadurch gekennzeichnet, daß die malignen Tumore Leber- oder Dickdarmtumore sind.

3. Verwendung nach Anspruch 1 oder 2, dadurch gekennzeichnet, daß die Glykokonjugate das Monosaccharid endständig oder mittelständig an ein pharmakologisch inertes Trägermolekül gebunden haben.

4. Verwendung nach den Ansprüchen 1 bis 3, dadurch gekennzeichnet, daß das Trägermolekül nicht selbst cytotoxisch gegen Tumorzellen wirksam ist.

5. Verwendung nach den Ansprüchen 1 bis 4, dadurch gekennzeichnet, daß als Glykokonjugat Arabinogalactan verwendet wird.

6. Verwendung nach den Ansprüchen 1 bis 5, dadurch gekennzeichnet, daß als Monosaccharid β-D-Galactose verwendet wird.

7. Verwendung nach den Ansprüchen 1 bis 6, dadurch gekennzeichnet, daß als Monosaccharid β-D-Galactose in einer Menge von 120 bis 150 g pro Tag verwendet wird.

## Claims

1. Use of monosaccharides which are specific for organ cell lectins and/or of glycoconjugates containing such monosaccharides for the preparation of a medicament for preventing metastases of malignant tumors from an organ-specific settling, wherein the monosaccharide is β-D-galactose, L-fucose, mannose, N-acetylglucosamine, N-acetylgalactosamine, N-acylneuraminic acid, or a mixture thereof.

2. The use according to claim 1, characterized in that the malignant tumors are hepatic tumors or colon tumors.

3. The use according to claim 1 or 2, characterized in that the glycoconjugates have the monosaccharide bonded to a pharmacologically inert carrier molecule in a terminal or central position.

4. The use according to claims 1 to 3, characterized in that carrier molecule itself is not cytoxically active against tumor cells.

5. The use according to claims 1 to 4, characterized in that arabinogalactan is used as the glycoconjugate.

6. The use according to claims 1 to 5, characterized in that β-D-galactose is used as the monosaccharide.

7. The use according to claims 1 to 6, characterized in that β-D-galactose is used as the monosaccharide in an amount of from 120 to 150 g per day.

## Revendications

1. Utilisation de monosaccharides, et/ou de glycoconjugués contenant des monosaccharides, spécifiques de la lectine organocellulaire, ledit monosaccharide étant du β-D-galactose, du L-fucose, du mannose, de la N-acetyl-glucosamine, de la N-acetyl-galactosamine, acide N-acyl-neuraminique ou un mélange desdits monosaccharides, pour produire un médicament empêchant la colonisation organo-spécifique de métastases de tumeurs malignes.

2. Utilisation selon la revendication 1, caractérisée en ce que les tumeurs malignes sont des tumeurs hépatiques ou coliques.

3. Utilisation selon la revendication 1 ou 2, caractérisée en ce que les glycoconjugués sont associés en bout ou en milieu de chaîne à une molécule vectrice pharmacologiquement inerte.

4. Utilisation selon les revendications 1 à 3, caractérisée en ce que la molécule vectrice n'est pas elle-même cytotoxiquement active contre les cellules tumorales.

5. Utilisation selon les revendications 1 à 4, caractérisée en ce que l'arabinogalactane est utilisé comme glycoconjugué.

6. Utilisation selon les revendications 1 à 5, caractérisée en ce que du β-D-galactose est utilisé comme monosaccharide.

7. Utilisation selon les revendications 1 à 6, caractérisée en ce que du β-D-galactose est utilisé comme monosaccharide selon un dosage de 120 à 150g par jour.
